# EUROPEAN PATENT APPLICATION

(11) **EP 1 656 947 A1**
(43) Date of publication of application: **17.05.2006**
(21) Application number: 04771259.1
(22) Date of filing: 05.08.2004
(51) Int. Cl.: A61K 38/08, A61K 39/00, A61K 39/39, A61P 35/00

(54) **PREVENTIVE AND/OR REMEDY FOR HEMATOPOIETIC TUMOR**

(30) Priority: 05.08.2003 JP 2003287208
(71) Applicant: Green Peptide Co., Ltd., Kurume-shi, Fukuoka 8300018 (JP)
(72) Inventor: ITOH, Kyogo, Saga 841-0205 (JP)
(74) Representative: Krauss, Jan
(86) International application number: PCT/JP2004/011232
(87) International publication number: WO 2005/011723

(57) **Abstract**

In the present invention, a useful molecular target for a specific immunotherapy of hematologic malignancies was found, and a means capable of preventing and/or treating hematologic malignancies was provided. Specifically, provided were the followings: an agent for preventing and/or treating hematologic malignancies comprising as an active ingredient at least one peptide having an amino acid sequence selected from any one of SEQ ID NOs: 1 to 10 in the sequence listing; the agent for preventing and/ or treating hematologic malignancies further comprising an adjuvant; and the preventing and/ or treating agent to use as a cancer vaccine for hematologic malignancies, which allowed prevention and/or treatment of hematologic malignancies such as an HLA-A24 positive hematologic malignancies or the hematologic malignancies that are HLA-A24 positive and express a protein containing the peptide.

## Description

### TECHNICAL FIELD

The present invention relates to an agent for preventing and/or treating hematologic malignancies which comprises a peptide as an active ingredient. More specifically, the present invention relates to an agent for preventing and/or treating hematologic malignancies which comprises as an active ingredient a peptide capable of inducing cytotoxic T lymphocytes. Further, the present invention relates to the aforementioned agent for preventing and/or treating hematologic malignancies which is used as a cancer vaccine for hematologic malignancies. Furthermore, the present invention also relates to a method for preventing and/or treating hematologic malignancies which comprises administering a peptide capable of inducing cytotoxic T lymphocytes.

### BACKGROUND ART

In the treatment of hematologic malignancies, nonspecific chemotherapies have been employed so far. In addition, therapies such as allogeneic bone marrow transplantation and autologous peripheral blood stem cell transplantation have been conducted. However, chemotherapies have the problem of accompanying adverse events, while allogeneic bone marrow transplantation and autologous peripheral blood stem cell transplantation have the problems of complications, graft rejection, and the difficulty of procuring donors. Further, these therapies are highly effective for younger people, but are not always effective for middle-aged and aged people whose generations are prone to cancers, because of the more possible side effects.

In recent years, molecular-targeting therapy which is based on the molecular mechanism of a malignant tumor has drawn attention as a therapy with fewer side effects. In such a therapy, a molecular targeting agent can act selectively and efficiently to cancer cells and be less harmful to normal cells, so that the therapy often allows obtaining high efficacy with fewer side effects. However, the therapy can not always provide high efficacy because malignant cells may sometimes acquire resistance to the drug.

Under these situations, a therapy highly effective for hematologic malignancies with fewer side effects has been desired to develop.

Immunotherapy has been developed as one of therapies for malignant tumors. For example, a specific immunotherapy using a tumor antigen peptide has been reported to be efficient against malignant tumors, especially malignant melanomas (Non-Patent References 1-6). In Europe and America, cancer vaccine therapies are being developed which comprises activating cytotoxic T-lymphocytes inside the body of a cancer patient by administration of a tumor antigen, and melanoma specific tumor antigens have been reported to provide successful results in clinical trials.

As an immunotherapy for hematologic malignancies, several treatment modalities have been proposed. For example, a vaccine therapy with irradiated autologous leukemia cells has been known. Further, Bcr/abl mutant antigen and PML/RARα mutant antigen has been considered to be the targets in T-cell immunotherapy against chronic myelogenous leukemia (which may be abbreviated as CML) and acute promyelocytic leukemia respectively (Non-Patent References 7 and 8). Furthermore, an immunoglobulin-derived peptide that is derived from B-cell malignant cells has considered to be possible target in T-cell response against the malignant cells (Non-Patent References 1 and 9). Leukemia-related antigens such as proteinase 3 in CML (Non-Patent Reference 10), ALK in lymphoma, and Wilms' suppressor gene WT1 in leukemia have also been reported to be available for a specific immunotherapy (Non-Patent References 8 and 11).

Immunotherapies against hematologic malignancies are reported to achieve tumor regression only in a few cases and cannot achieve tumor specific immune responses in most of cancer patients, and thus the clinical efficacy thereof is currently limited.

The References cited in the specification are listed as follows:
Patent Reference 1: "International Publication No. WO 01/011044 pamphlet"
Non-patent Reference 1: Bendandi, M. et al., "Nature Medicine", 1999, Vol.5, p.1171-1177.
Non-patent Reference 2: Trojan, A. et al., "Nature Medicine", 2000, Vol.6, p.667-672. Non-patent Reference 3: Kikuchi, M. et al., "International Journal of Cancer", 1999, Vol.81, p.459-466.
Non-patent Reference 4: Yang, D. et al., "Cancer Research", 1999, Vol.59, p.4056-4063.
Non-patent Reference 5: Nakao, M. et al., "Journal of Immunology", 2000, Vol. 164, p.2565-2574.
Non-patent Reference 6: Nishizaka, S. et al., "Cancer Research", 2000, Vol.60, p.4830-4837.
Non-patent Reference 7: Pinilla-Ibarz, J. et al., "Blood", 2000, Vol.95, p.1781-1787.
Non-patent Reference 8: Appelbaum, F.R., "Nature", 2001, Vol.41 1, p.385-389.
Non-patent Reference 9: Hsu, F.J. et al., "Blood", 1996, Vol.89, p.3129-3135.
Non-patent Reference 10: Molldrem, J. et al., "Blood", 1997, Vol.88, p.2450-2457.
Non-patent Reference 11: Passoni, L. et al., "Blood", 2002, Vol.99, p.2100-2106.
Non-patent Reference 12: Harashima, N. et al., "European Journal of Immunology", 2001, Vol.31, p.323-332.
Non-patent Reference 13: Miyagi, Y et al., "Clinical Cancer Research", 2001, Vol.7, p.3950-3962.
Non-patent Reference 14: Ito, M. et al., "Cancer Research", 2001, Vol.61, p.2038-2046.

### DISCLOSURE OF THE INVENTION

### [PROBLEM TO BE SOLVED BY THE INVENTION]

An object of the present invention is to identifying molecular targets useful in an immunotherapy for hematologic malignancies and to provide a method for preventing and/or treating hematologic malignancies.

### [MEANS FOR SOLVING THE OBJECT]

The present inventor has already identified and reported a series of epithelial cancer-related antigens and peptides derived from the antigens which can induce peptide-specific and tumor-reactive cytotoxic T lymphocytes from peripheral blood mononuclear cells (hereinafter abbreviated to PBMCs) of an epithelial cancer patient (Patent Reference1; Non-Patent References 3-6 and 12). Further, the inventor disclosed that vaccination using these peptides was effective in enhancing the immunocompetence of a variety of epithelial cancer patients and in achieving clinical efficacy (Non-Patent Reference 13).

The present inventor strenuously conducted studies to solve the above-described problem and found that some of the epithelial cancer-related antigens reported previously are expressed in hematologic malignant cell lines, and that a peptide derived from the antigen can induce by vaccination thereof to a patient with hematologic malignancy a cytotoxic T lymphocyte being reactive to a malignant tumor cell, and thus completed the present invention. There is a report showing that a leukemia-related antigen induced a cytotoxic T lymphocyte from PBMCs derived from a patient with hematologic malignancy (Non-Patent Reference 10), but there is no report showing the induction of a cytotoxic T lymphocyte by an epithelial cancer-related antigen in a patient with hematologic malignancy and the efficacy of the peptide vaccine.

Thus, the present invention relates to an agent for preventing and/or treating hematologic malignancies, which comprises as an active ingredient at least one peptide having an amino acid sequence selected from the group consisting of SEQ ID NOs: 1, 2, 3, 4, 5, 6, 7, 8, 9 and 10 in the sequence listing.

The present invention further relates to an agent for preventing and/or treating hematologic malignancies, which comprises as an active ingredient at least one peptide having an amino acid sequence selected from the group consisting of SEQ ID NOs: 1, 2, 4, 5, 8 and 10 in the sequence listing.

The present invention still further relates to an agent for preventing and/or treating hematologic malignancies, which comprises an agent for preventing and/or treating hematologic malignancies comprising as an active ingredient at least one peptide having an amino acid sequence selected from the group consisting of SEQ ID NOs: 1, 2, 4, 5, 8 and 10 in the sequence listing, and further comprises as an active ingredient at least one peptide having an amino acid sequence selected from the group consisting of SEQ ID NOs: 3, 6, 7 and 9 in the sequence listing,

The present invention still further relates to an agent for preventing and/or treating hematologic malignancies, which comprises as an active ingredient at least one peptide having an amino acid sequence selected from the group consisting of SEQ ID NOs: 1, 2 and 4 in the

### sequence listing.

The present invention still further relates to an agent for preventing and/or treating hematologic malignancies, comprising as an active ingredient at least one peptide having an amino acid sequence selected from the group consisting of SEQ ID NOs: 2, 4 and 10 in the

### sequence listing.

The present invention still further relates to an agent for preventing and/or treating hematologic malignancies, comprising as an active ingredient at least one peptide having an amino acid sequence selected from the group consisting of SEQ ID NOs: 5 and 8 in the sequence listing.

The present invention still further relates to the aforementioned agent for preventing and/or treating hematologic malignancies, which further comprises an adjuvant.

The present invention still further relates the aforementioned preventing and/or treating agent, wherein the hematologic malignancies are hematologic malignancies having HLA-A24 molecules on their cell surface.

The present invention still further relates to the aforementioned preventing and/or treating agent, wherein the hematologic malignancies are hematologic malignancies which have HLA-A24 molecules on their cell surface and express a protein containing the peptide that is an active ingredient for the preventing and/or treating agent.

The present invention still further relates to the aforementioned agent for preventing and/or treating hematologic malignancies, wherein the agent is used as a cancer vaccine for the hematologic malignancies.

The present invention still further relates to a medicament for preventing and/or treating hematologic malignancies having HLA-A24 molecules on their cell surface, which comprises at least one peptide having an amino acid sequence selected from the group consisting of SEQ ID NOs: 1, 2, 4, 5, 8 and 10 in the sequence listing and an adjuvant.

The present invention still further relates to a method for preventing and/or treating hematologic malignancies that have HLA-A24 molecules on their cell surface, which comprises administering a therapeutically effective amount of at least one peptide having an amino acid sequence selected from the group consisting of SEQ ID NOs: 1, 2, 4, 5, 8 and 10 in the sequence listing together with an adjuvant.

### [ADVANTAGE OF THE INVENTION]

The present invention can provide an agent for preventing and/or treating hematologic malignancies comprising a peptide as an active ingredient which is derived from an epithelial cancer-related antigen. In one embodiment of use, the agent for preventing and/or treating hematologic malignancies according to the present invention may be used as a cancer vaccine. The agent for preventing and/or treating hematologic malignancies according to the present invention can induce cytotoxic T lymphocytes targeting for tumor in a patient with hematologic malignancy, and thus may be used in a specific immunotherapy for hematologic malignancies. For example, it may be useful in preventing and/or treating HLA-A24 positive hematologic malignancies and also HLA-A24 positive hematologic malignancies expressing a protein that contains the peptide being an active ingredient of the preventing and/or treating agent. The HLA-A24 allele is frequently found in many races. Approximately 60% of Asian population, approximately 20% of Caucasian, and approximately 40% of Hispanic and black are known to have this allele. Therefore, the present invention can be expected to be useful for many patients with hematologic malignancies. Moreover, no or few side effects are observed regarding the agent for preventing and/or treating hematologic malignancies according to the present invention. Thus, also from this point of view, the agent for preventing and/or treating hematologic malignancies according to the present invention may be effective as a preventing and/or treating means.

### BRIEF DESCRIPTION OF THE DRAWINGS

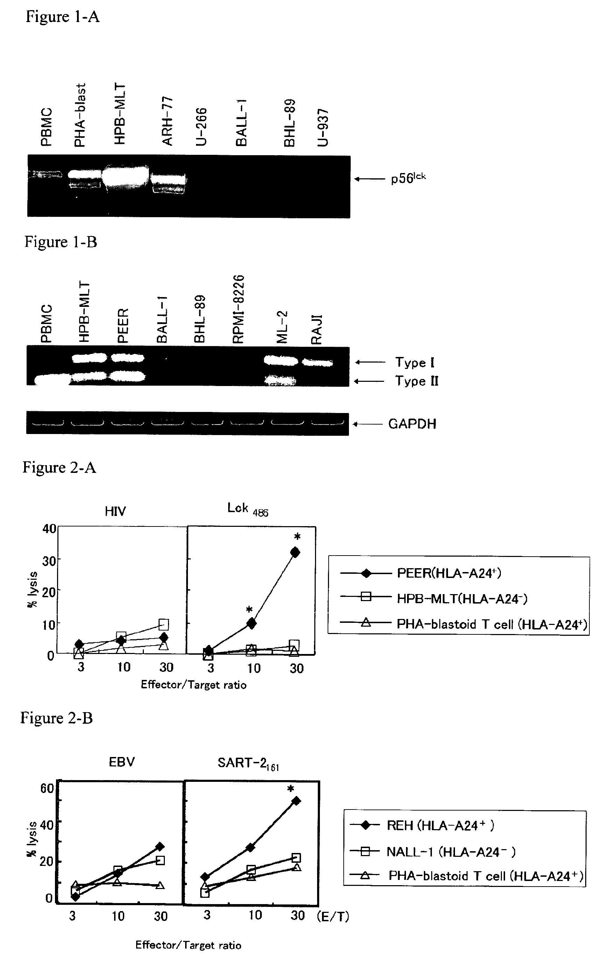

Fig. 1-A illustrates the results showing the expression of p56^{lck} protein in a panel of cell lines, which was detected by Western blot analysis using anti-p56^{lck} monoclonal antibody. The high expression was observed in HPB-MLT, an adult T-cell leukemia cell line, and ARH-77, a myeloma cell line. The expression was also observed in PBMCs and phytohaemagglutinin (PHA) blastoid T-cells both of which are normal cells. (Example 2)
Fig. 1-B illustrates the results showing the expression of two different promoter transcripts of lck gene in a panel of cell lines, which was detected by RT-PCR. The promoter transcripts of both type I and type II were observed in HPB-MLT and PEER, the adult T-cell leukemia cell lines, and ML-2, a myelogenous leukemia cell line. Only the promoter transcript of the type I was observed in RAJI, a Burkitt lymphoma cell line, BALL-1, a B-cell leukemia cell line, BHL-89, a lymphoma cell line, and RPMI-8226, a multiple myeloma cell line, while only the promoter transcript of the type II was observed in PBMC, a normal cell. The expression of GAPDH (glyceraldehyde-3-phosphate dehydrogenase) was measured as a control for RT-PCR (Example 2).

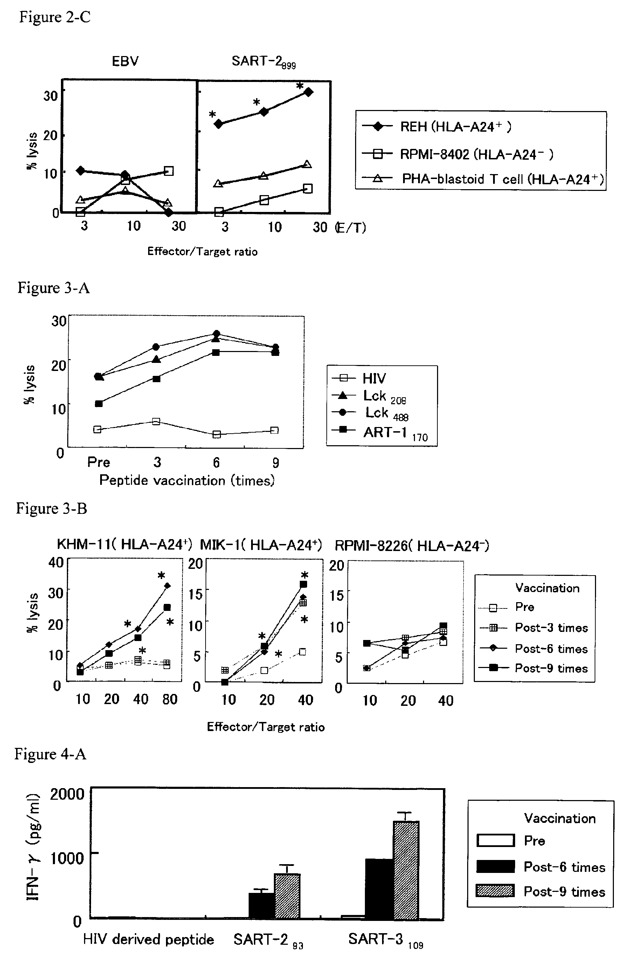

Fig. 2-A illustrates that PBMCs derived from a patient with an adult T-cell leukemia (ATL) (patient No. 8 in table 2), which were subjected to the in vitro stimulation using a peptide Lck₄₈₆ (SEQ ID NO: 3), exhibited cytotoxic activity (in the figure, shown by % lysis) against PEER, an HLA-A24⁺ATL cell line, but did not exhibit cytotoxic activity against an HLA-A24⁻ tumor cell line and an HLA-A24⁺PHA blastoid T-cell that is a normal cell (right panel). On the other hand, PBMCs stimulated with a human immunodeficiency virus (HIV)-derived peptide (SEQ ID NO: 11) exhibited no cytotoxic activity (left panel). (Example 3)
Fig. 2-B illustrates that PBMCs derived from a patient with chronic lymphocytic leukemia (patient No. 2 in table 2), which were subjected to the in vitro stimulation using a peptide SART-2₁₆₁ (SEQ ID NO: 6), exhibited cytotoxic activity (in the figure, shown by % lysis) against REH, an HLA-A24⁺ATL cell line, but did not exhibit cytotoxic activity against an HLA-A24⁻ tumor cell line and an HLA-A24⁺ PHA blastoid T-cell that is a normal cell (right panel). On the other hand, PBMCs stimulated with an Epstein-Barr virus (EBV)-derived peptide (SEQ ID NO: 12) exhibited no cytotoxic activity (left panel). (Example 3)
Fig. 2-C illustrates that PBMCs derived from a patient with non-Hodgkin's lymphoma (patient No. 4 in table 2), which were subjected to the in vitro stimulation using a peptide SART-2₁₆₁ (SEQ ID NO: 7), exhibited cytotoxic activity (in the figure, shown by % lysis) against REH, an HLA-A24⁺ATL cell line, but did not exhibit cytotoxic activity against an HLA-A24⁻ tumor cell line and an HLA-A24⁺PHA blastoid T-cell that is a normal cell (right panel). On the other hand, PBMCs stimulated with an EBV-derived peptide (SEQ ID NO: 12) exhibited no cytotoxic activity (left panel). (Example 3)
Fig. 3-A illustrates that PBMCs derived from a patient with multiple myeloma (patient No. 6 in table 2), which were incubated with Lck₂₀₈ (SEQ ID NO: 1), Lck₄₈₈ (SEQ ID NO: 2) or ART-1₁₇₀ (SEQ ID NO: 10), exhibited enhanced induction of cytotoxic activity (in the figure, shown by % lysis) against C1R-A24 cells pulsed with corresponding each peptide, with the increasing number of peptide vaccination. (Example 4)
Fig. 3-B illustrates that PBMCs derived from a patient with multiple myeloma (patient No. 6 in table 2) exhibited enhanced cytotoxic activity (in the figure, shown by % lysis) against KHM-11 and MIK-1, the HLA-A24⁺ multiple myeloma cell lines, with the increasing number of peptide vaccination. On the other hand, cytotoxic activity against RPMI-8226, an HLA-A24⁻ multiple myeloma cell line, was not observed even after the peptide vaccination. (Example 4)

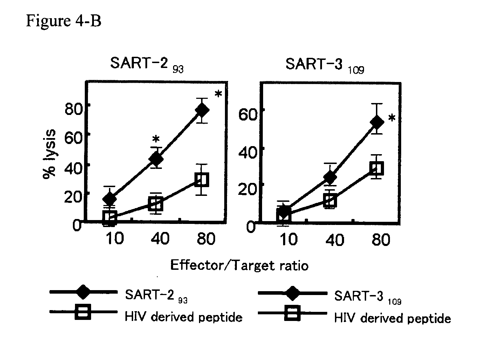

Fig. 4-A illustrates that PBMCs derived from a patient with chronic lymphocytic leukemia (patient No. I in table 2) exhibited enhanced production of interferon (IFN)-γ in response to C1R-A24 cells pulsed with SART-2₉₃ (SEQ ID NO: 5) or SART-3₁₀₉ (SEQ ID NO: 8), with the increasing number of peptide vaccination (Example 5).
Fig. 4-B illustrates that PBMCs derived from a patient with chronic lymphocytic leukemia (patient No. 1 in table 2) after vaccination exhibited a higher cytotoxic activity against C1R-A24 cells pulsed with SART-2₉₃ (SEQ ID NO: 5) or SART-3₁₀₉ (SEQ ID NO: 8) than against the cells pulsed with a HIV-derived peptide (Example 5).

### DETAILED DESCRIPTION OF THE INVENTION

The present invention claims priority from Japanese Patent Application No. 2003-287208, which is incorporated herein by reference.

One aspect of the present invention relates to an agent for preventing and/or treating hematologic malignancies which is characterized by the function of inducing anti-tumor immune responses against hematologic malignancy in a test subject.

Hematologic malignancies are tumors developed in hematopoietic organs such as bone marrow and lymphoid tissues. Hematologic malignancies include malignant tumors such as acute myelogenous leukemia, acute lymphocytic leukemia, chronic myelogenous leukemia, chronic lymphocytic leukemia, malignant lymphoma, and multiple myeloma.

Test subjects herein include patients with hematologic malignancies, bloods collected from patients with hematologic malignancies, and leukocyte fractions prepared from bloods of patients with hematologic malignancies.

The anti-tumor immune responses mean immune responses caused against a tumor, which are primarily associated with cytotoxic T lymphocytes. Therefore, the present invention provides an agent for preventing and/or treating hematologic malignancies which is characterized by the function of inducing cytotoxic T lymphocytes against hematologic malignancies in a test subject.

The agent for preventing and/or treating hematologic malignancies means, for example, a medicament having an effect to prevent the development or recurrence of hematologic malignancies, a medicament having an effect to inhibit the progression and aggravation of hematologic malignancies, and a medicament having an efficacy to improve or cure the disease condition of a hematologic malignancies such as the reduction of leukocyte count in blood.

In one aspect, the agent for preventing and/or treating hematologic malignancies according to the present invention comprises as an active ingredient an effective amount of one or more peptides each of which has an amino acid sequence selected from the group consisting of SEQ ID NOs: 1, 2, 3, 4, 5, 6, 7, 8, 9 and 10. More preferably, it is an agent for preventing and/or treating hematologic malignancies comprising as an active ingredient an effective amount of one or more peptides each of which has an amino acid sequence selected from the group consisting of SEQ ID NOs: 1, 2, 4, 5, 8 and 10. It may be an agent for preventing and/or treating hematologic malignancies which comprises one or more peptides each of which has an amino acid sequence selected from the group consisting of SEQ ID NOs: 3, 6, 7 and 9 in addition to one or more peptides each of which has an amino acid sequence selected from the group consisting of SEQ ID NOs: 1, 2, 4, 5, 8 and 10 More preferably, it is an agent for preventing and/or treating hematologic malignancies comprising as an active ingredient an effective amount of one or more peptides each of which has an amino acid sequence selected from the group consisting of SEQ ID NOs: 1, 2 and 4. Further, it may be an agent for preventing and/or treating hematologic malignancies comprising as an active ingredient an effective amount of one or more peptides each of which has an amino acid sequence selected from the group consisting of SEQ ID NOs: 2, 4 and 10. Further, it may be an agent for preventing and/or treating hematologic malignancies comprising as an active ingredient an effective amount of one or more peptides each of which has an amino acid sequence selected from the group consisting of SEQ ID NOs: 5 and 8.

Any peptide having each amino acid sequence set forth in SEQ ID NOs: 1, 2, 3, 4, 5, 6, 7, 8, 9 and 10 is a peptide derived from an epithelial cancer-related antigen (hereinafter, referred to as an epithelial cancer-related antigen peptide). The peptides having each amino acid sequence set forth in SEQ ID NOs: 1, 2 and 3 are p56^{lck} protein-derived peptides, and hereinafter may be referred to as Lck208, Lck488 and Lck486, respectively (Non-Patent Reference 12). The peptide having an amino acid sequence set forth in SEQ ID NO: 4 is a SART-1-derived peptide, and hereinafter may be referred to as SART-1₆₉₀ (Non-Patent Reference 3). The peptides having each amino acid sequence set forth in SEQ ID NOs: 5, 6 and 7 are SART-2-derived peptides, and hereinafter may be referred to as SART-2₉₃, SART-2₁₆₁ and SART-2₈₉₉, respectively (Non-Patent Reference 5). The peptides having each amino acid sequence set forth in SEQ ID NOs: 8 and 9 are SART-3-derived peptides, and hereinafter may be referred to as SART-3₁₀₉ and SART-3₃₁₅, respectively (Non-Patent Reference 4). The peptide having an amino acid sequence set forth in SEQ ID NO: 10 is an ART-1-derived peptide, and hereinafter may be referred to as ART-1₁₇₀ (Non-Patent Reference 6). Each peptide is represented herein by both the name of the protein from which the peptide is derived and a number showing the position of the N-terminal amino acid residue of the peptide in the amino acid sequence of the protein. For example, Lck208 means that it is an Lck protein-derived peptide and that the N-terminal amino acid residue of the peptide is an amino acid residue located at the 208^{th} position in the amino acid sequence of the protein.

In the present invention, it was found that vaccination of the aforementioned peptide to a patient with hematologic malignancy can induce cytotoxic T lymphocytes in the patient in an HLA-A24 restricted-manner which are targeted to hematologic malignant cells, and thereby it can achieve a clinical efficacy in conjunction with a decrease of malignant cells. It has already been reported that the aforementioned peptides can induce the peptide-specific cytotoxic T lymphocytes from PBMCs of an epithelial cancer patient in an HLA-A24 restricted-manner (Non-Patent References 3-6 and 12). However, it has not been reported that these peptides can induce peptide-specific cytotoxic T lymphocytes from PBMCs of a patient with hematologic malignancy.

Vaccination with a peptide means the administration of a peptide for the purpose of inducing and/or enhancing peptide-specific immune responses in a subject to be vaccinated.

The term "to induce a peptide-specific cytotoxic T lymphocyte in an HLA-A24 restricted-manner" means "to induce a cytotoxic T lymphocyte that recognizes a particular peptide presented by a human leukocyte antigen molecule, a major histocompatibility complex molecule, which is expressed on a cell surface, but scarcely or never recognizes the other peptide presented likewise, by a peptide presented by an HLA class I molecule of which HLA class I phenotype is A24".

Specifically, in a case of a patient with multiple myeloma patient (see Example 4), six times vaccination was conducted with the combination of three kinds of vaccines prepared by mixing each of the peptides Lck488 (SEQ ID NO: 2), SART-1₆₉₀ (SEQ ID NO: 4) and ART-1₁₇₀ (SEQ ID NO: 10) with an adjuvant respectively. Then, the patient was subsequently vaccinated with the combination of three kinds of vaccines prepared by mixing each of the peptides Lck208 (SEQ ID NO: 1), Lck488 (SEQ ID NO: 2) and SART-1₆₉₀ (SEQ ID NO: 4) with an adjuvant respectively. In the vaccination conducted with the combination of the peptides, cytotoxic T lymphocytes specific to each peptide increased in PBMCs of the patient, with the increasing number of peptide vaccination (see Figure 3-A).

In another clinical trial case (see Example 6) of a patient with chronic lymphocytic leukemia, vaccination was conducted with two kinds of vaccines prepared by mixing each of the peptides SART-2₉₃ (SEQ ID NO: 5) and SART-3₁₀₉ (SEQ ID NO: 8) with an adjuvant respectively. In this clinical case, cytotoxic T lymphocytes specific to each peptide increased in PBMCs of the patient, with the increasing number of peptide vaccination (see Figures 4-A and 4-B).

Since cytotoxic T lymphocytes specific to each peptide increased even in a case of vaccination with combination of peptides, it is believed that even the vaccination with each peptide alone would induce peptide-specific cytotoxic T lymphocytes and further provide clinical efficacy likewise.

The adjuvant contained in the vaccine together with the peptide in vaccination was used to enhance the induction of anti-tumor immune responses by the peptide. The peptides, which are the active ingredients to induce anti-tumor immune responses, were able to induce cytotoxic T lymphocytes in vitro when used alone, which suggests that even a medicament consisting of only the peptide may be efficient as an agent for preventing and/or treating hematologic malignancies. Since an adjuvant contained together can provide better anti-tumor effects, the use of the adjuvant is recommended.

Any peptide having each amino acid sequence set forth in SEQ ID NOs: 1, 2, 4, 5, 8 and 10 which was used in the vaccination induced cytotoxic T lymphocytes from PBMCs derived from a variety of pre-vaccinated patients with hematologic malignancies in an HLA-A24 dependent manner. Besides these peptides, Lck486 (SEQ ID NO: 3), SART-2₁₆₁ (SEQ ID NO: 6), SART-2₈₉₉ (SEQ ID NO: 7) and SART-3₃₁₅ (SEQ ID NO: 9) induced cytotoxic T lymphocytes from PBMCs of patients with hematologic malignancies in an HLA-A24 dependent manner. From these facts, a peptide having each amino acid sequence set forth in SEQ ID NOs: 3, 6, 7 and 9 can be used as an active ingredient for an agent for preventing and/or treating hematologic malignancies.

Consequently, a peptide having an amino acid sequence selected from the group consisting of SEQ ID NOs: 1, 2, 3, 4, 5, 6, 7, 8, 9 and 10 may be used either alone or in combination of two or more as an active ingredient of the agent for preventing and/or treating hematologic malignancies according to the present invention.

In one aspect of the use, the agent for preventing and/or treating hematologic malignancies according to the present invention may be used as a cancer vaccine. The cancer vaccine herein means a drug which induces and/or enhances immune responses specific to malignant cells; activates cytotoxic T lymphocytes in the body of a cancer patient; and then selectively damages malignant cells.

For example, when using the aforementioned peptides as a cancer vaccine, vaccination can be conducted not only with a cancer vaccine that contains all of the selected several kind of peptides, but also with several cancer vaccines each of which contains each of the peptides respectively.

The agent for preventing and/or treating hematologic malignancies according to the present invention may contain an adjunct that can enhance anti-tumor immune responses. Examples of such an adjunct include interleukin-2 that is effective to expand cytotoxic T lymphocytes. The agent for preventing and/or treating hematologic malignancies according to the present invention, which contains adjuncts such as adjuvants and carriers, may provide higher anti-tumor effects, when using as a cancer vaccine. An adjuvant may be used either alone or in combination of two or more. Examples of adjuvant include Freund's complete adjuvant, alum, lipid A, monophosphoryl lipid A, bacterial preparation such as BCG (Bacillus-Calmette-Guerrin) preparation, bacterial component preparation such as tuberculin preparation, natural polymer product such as keyhole limpet hemocyanin and yeast mannan, muramyl tripeptide, muramyl dipeptide or derivatives thereof, alum, non-ionic block copolymers. Montanide ISA-51 was used in the examples herein. Useful adjuvants are not limited to these specific examples, and any other material may be used as long as it can enhance anti-tumor immune responses. It can be determined whether an adjuvant should be used or not, by using indicators such as the intensity of inflammatory response at a vaccination site, the anti-tumor efficacy produced by vaccination, and the intensity of cytotoxic activity of peripheral blood mononuclear cells from a test subject. Carrier is not particularly limited as long as it is not harmful to human body and may lead an enhanced antigenicity, which is exemplified by cellulose, polymerized amino acid, and albumin.

The agent for preventing and/or treating hematologic malignancies according to the present invention can be specifically exemplified by an agent for preventing and/or treating hematologic malignancies comprising one or more peptides each of which has an amino acid sequence selected from the group consisting of SEQ ID NOs: 1, 2, 4, 5, 8 and 10 in the sequence listing and an adjuvant.

The agent for preventing and/or treating hematologic malignancies according to the present invention may comprise, besides the aforementioned peptides, an effective amount of the other anti-tumor peptide that can induce anti-tumor immune responses against hematologic malignancies. The other anti-tumor peptide for addition may be a peptide that can induce cytotoxic T lymphocytes in an HLA-A24 dependent manner or a peptide that can induce cytotoxic T lymphocytes in an HLA class I dependent manner which phenotype is other than HLA-A24. The other anti-tumor peptide is preferably an anti-tumor peptide that can induce peptide-specific cytotoxic T lymphocytes from PBMCs of a patient to whom the preventing and/or treating agent of the present invention is applied. Evaluation of specific cytotoxic T lymphocytes induction by a peptide can be carried out by culturing PBMCs with the peptide for a suitable period, and then reacting them with cells having an HLA class I molecule on their cell surface which are previously pulsed with the corresponding peptide, and finally measuring the amount of interferon-γ (IFN-γ) production (see Example 3). For example, increased amount of IFN-γ production in comparison with that from PBMCs cultured in the absence of the peptide indicates the induction of cytotoxic T lymphocytes.

The hematologic malignancies to which the agent for preventing and/or treating hematologic malignancies according to the present invention may be applied are preferably HLA-A24 positive hematologic malignancies, and more preferably hematologic malignancies that exhibit the expression of one or more kinds of protein containing the peptide that is an active ingredient of the agent, such as p56^{lck} protein, SART-1, SART-2, SART-3 and ART-1. "HLA-A24 positive (also referred to as HLA-A24⁺)" means expressing an HLA-A24 molecule on cell surface. A tumor is an autologous cell that has acquired abnormal proliferative activity for some reasons. Therefore, in human having an HLA class I phenotype of HLA-A24, HLA-A24 molecules are in most cases expressed on the surfaces of the cells, irrespective of the cells being normal or tumoral. The HLA class I phenotype may be determined in accordance with the well known methods using, for example, peripheral blood mononuclear cells or a part of the hematologic malignancy collected from a patient with hematologic malignancy (Non-Patent-Reference 5). Alternatively and simply, it can be determined by the conventional serological methods using blood that is collected from a patient with hematologic malignancy (Non-Patent-Reference 4). The expression of a protein in a various kinds of hematologic malignancy can be detected with a part of the hematologic malignancy collected from a patient with hematologic malignancy, for example, by the Western blot analysis using an antibody against a protein in interest to detect, or by the detection of the promoter transcript of a gene encoding a protein in interest by using the reverse transcription-polymerase chain reaction (RT-PCR).

The specific examples of hematologic malignancies include adult T-cell leukemia (sometimes abbreviated as ATL); T-cell acute lymphocytic leukemia (sometimes abbreviated as T-ALL); T-cell lymphoma; myelogenous leukemia; multiple myeloma (sometimes abbreviated as MM); myelodysplastic syndrome (sometimes abbreviated as MDS); acute monocytic leukemia; B-cell acute lymphocytic leukemia (sometimes abbreviated as B-ALL); B-cell lymphoma; Burkitt lymphoma; chronic lymphocytic leukemia (sometimes abbreviated as CLL); mantle cell lymphoma (sometimes abbreviated as MCL); and non-Hodgkin's lymphoma (sometimes abbreviated as NHL), but are not limited to these specific examples. The agent for preventing and/or treating hematologic malignancies according to the present invention may be applied preferably to MM, MDS, ATL, T-ALL, or CLL, and more preferably to MM or CLL.

The peptides may be produced by a common method for chemical synthesis of amino acids. Examples of the method for chemical synthesis include a peptide synthesis method using an ordinary liquid phase method and solid phase method, such as the Fmoc method. Alternatively, an amino acid synthesizer that is commercially available may also be used for the production. Further, the peptides may be obtained by using a genetic engineering technique. For example, the peptides may be produced by preparing a recombinant DNA (expression vector) that can express a gene encoding the peptide in interest in a host cell, transfecting it to a suitable host cell such as E. coli, culturing the transformant, and then collecting the peptide in interest from the resulting culture product.

The agent for preventing and/or treating hematologic malignancies according to the present invention may contain adjuncts such as an adjuvant, besides the peptide that an active ingredient of the agent, and further it may contain one or more pharmaceutical acceptable carriers. A useful pharmaceutical carrier is exemplified by a stabilizer, bactericide, buffer, isotonic agent, chelating agent, pH adjuster, surfactant, fillers, extender, binder, wetting agent, disintegrant, lubricant, analgesic agent, diluent, excipient and the like which are suitably selected and used in accordance with the administration form of the preparation to be obtained.

A preparation form may be selected according to an administration form. Typical examples of the preparation form include a liquid formulation, emulsion, liposome formulation, fat emulsion, clathrate like cyclodextrin, suspension, ointment formulation, cream formulation, transdermal absorption formulation, transmucosal absorption formulation, pills, tablets, pills, capsules, powder, powdered drug, fine granule, granule, syrup and the like, but are not limited to these examples. These can be further classified according to the administration route into oral formulation, parenteral formulation, nasal formulation, transvaginal formulation, suppositorial formulation, sublingual agents, inhalant formulation, eye drop formulation, ear drop formulation and the like, which can be respectively blended, formed and prepared according to conventional methods. Further, a liquid formulation also may be lyophilized so as to keep it in good state in preservation, which is then dissolved in water, a buffered solution containing physiological saline and the like, and so on to have a suitable concentration at the time of using.

Injectable solutions can be prepared using a carrier comprising a salt solution, a glucose solution or a mixture of salt water and a glucose solution.

Liposome formulation can be prepared in the following manner: by dissolving the substance of interest in a solvent (e.g., ethanol) to make a solution, adding a solution of phospholipids dissolved in an organic solvent (e.g., chloroform), removing the solvent by evaporation and adding a phosphate buffer thereto, agitating the solution and then subjecting it to sonication followed by centrifugation to obtain supernatant, and finally, filtrating the supernatant for recovering a liposome.

Fat emulsion can be prepared in the following manner: by mixing the substance of interest, an oil ingredient (vegetable oil such as soybean oil, sesame oil, olive oil, or MCT), an emulsifier (such as phospholipid), and the like; heating the mixture to make a solution; adding water of a required quantity; and then emulsifying or homogenizing by use of an emulsifier (homogenizer, e.g., a high pressure jet type, an ultrasonic type, or the like). The fat emulsion may be also lyophilized. For conducting lipid-emulsification, an auxiliary emulsifier may be added, and examples thereof include glycerin or saccharides (e.g., glucose, sorbitol, fructose, etc.).

Inclusion into a cyclodextrin formulation may be carried out in the following manner: by dissolving the substance of interest in a solvent (e.g., ethanol); adding a solution of cyclodextrin dissolved in water under heating thereto; chilling the solution and filtering the precipitates; and drying under sterilization. At this time, the cyclodextrin to be used may be appropriately selected from among those having different void sizes (α, β, or γ type) in accordance with the bulkiness of the substance.

A suspension can be prepared using water, saccharides such as sucrose, sorbitol, or fructose, glycols such as PEG, and oils.

The other preparation forms may be prepared likewise by an ordinary method.

The amount of an active ingredient contained in a formulation and the dose range thereof are not particularly limited, and can be determined according to the following: the efficacy of a peptide; administration form; the type of disease; the characteristics of the subject (e.g., body weight, age, symptomatic conditions and whether being taking other medicament); and the judgment of the physician in charge. In general, a suitable dosage may fall, for example, within a range of about 0.01 µg to 100 mg per dose, preferably about 0.1 µg to 10 mg per dose, more preferably 1 µg to 1 mg per dose. However, a dosage may be altered using conventional experiments for optimization that are well known in the art.

In terms of administration form, either local administration or systemic administration may be selected. In both occasion, suitable administration form may be selected in accordance with a disease, symptomatic conditions, or other factors. Systemic administration may be carried out, for example, by oral administration, intravenous administration and intraarterial administration. Local administration may be carried out, for example, by subcutaneous administration, intradermal administration and intramuscular administration.

Alternatively, an effective anti-tumor effects can be obtained also by collecting a fraction of mononuclear cells from the peripheral blood of a patient, incubating them with the agent for preventing and/or treating hematologic malignancies of the present invention, and then returning the fraction of mononuclear cells in which induction of CTL and/or activation of CTL was observed, into the blood of the patient. Culture conditions, such as mononuclear cell concentration, concentration of the agent for preventing and/or treating hematologic malignancies, culture time, and the like, can be determined by simply repeating experiments. A substance having a capability to enhance the growth of lymphocytes, such as interleukin-2, may be added during culturing.

Vaccination may be preferably conducted by local administration by means of an intradermal injection, subcutaneous injection or intramuscular injection. The administration may be conducted by dividing the aforementioned dosage to administer once to several times a day. For vaccination, single administration may be conducted, but repeating administration to the same site or different sites is preferred. For example, periodic administration once every week or once every one to several months can be employed. More specifically, the administration may be conducted once every two weeks for a long period of one year or more. A cancer vaccine containing no adjuvant may be administered itself alone, but may be administered with administering an adjuvant at the same site. A cancer vaccine containing an adjuvant may be directly used for local administration. In terms of a vaccination protocol, a suitable protocol for each case can be designed by taking account of dosage and administration period suitable for the symptoms of a test subject with observing the efficacy of vaccination.

Another aspect of the present invention is a method for preventing and/or treating hematologic malignancies, comprising the administration of a therapeutically effective amount of one or more peptides each of which has an amino acid sequence selected from the group consisting of SEQ ID NOs: 1, 2, 3, 4, 5, 6, 7, 8, 9 and 10. More preferably, the peptide administered is a peptide each of which has an amino acid sequence selected from the group consisting of SEQ ID NOs: 1, 2, 4, 5, 8 and 10 in the sequence listing. More preferably, each of these peptides is administered together with an adjuvant. Further, two or more of these peptides may be administered in combination together with an adjuvant. The hematologic malignancies to which the method for preventing and/or treating hematologic malignancies according to the present invention may be applied are preferably HLA-A24 positive hematologic malignancies, and more preferably hematologic malignancies that exhibit the expression of one or more kinds of protein containing the peptide that is an active ingredient of the agent, such as p56^{lck} protein, SART-1, SART-2, SART-3 and ART-1.

Hereinafter, the present invention may be explained more particularly with examples; however, the present invention is not limited to the following examples.
All experiments described in the Examples were conducted after obtaining written informed consent from patients who provided samples. The clinical study of peptide vaccine therapy was conducted in accordance with a clinical protocol (protocol No: 2031) which was approved by the Institutional Ethical Review Board of Kurume University after obtaining written informed consent from patients.

### EXAMPLE 1

(Peptide preparation used for vaccination: 1)
The peptides used in a clinical study were the followings: p56^{lck} protein-derived peptides Lck₂₀₈ (SEQ ID NO: 1) and Lck₄₈₈ (SEQ ID NO: 2); SART-1-derived peptides SART-1₆₉₀ (SEQ ID NO: 4); and ART-1-derived peptides ART-1₁₇₀ (SEQ ID NO: 10). These peptides were prepared under conditions of Good Manufacturing Practice by Multiple Peptide System Inc.

For an adjuvant, Montanide ISA-51 adjuvant (Seppic, Inc.) was used.

Three mg of each peptide with sterile physiological saline added in a 1:1 volume to the Montanide ISA-51, and mixed in a Vortex mixer to prepare an emulsion. Thus, four kinds of emulsions were prepared, each of which contains Lck₂₀₈ (SEQ ID NO: 1), Lck₄₈₈ (SEQ ID NO: 2), SART-1₆₉₀ (SEQ ID NO: 4) or ART-1₁₇₀ (SEQ ID NO: 10).

### EXAMPLE 2

(Measurement of expression of epithelial cancer-related antigens in hematologic malignancies)
The expression of epithelial cancer-related antigens (p56^{lck} proteins, ART-1, SART-1, SART-2 and SART-3) in a panel of hematologic malignant cell lines was studied. The following cell lines were used in the study: B-cell acute lymphocytic leukemia (B-ALL) cell lines (REH, HALL1, NALM6, NALM16, KOPN-K, BALM1-2 and BALL-1); T-cell acute lymphocytic leukemia (T-ALL) cell lines (KOPT, RPMI-8402, CCRF-CEM, HPB-ALL, MOLT-4, CCRF-HSB-2 and PEER); acute myelogenous leukemia cell lines (ML1, ML2, ML3 and KG1); acute monocytic leukemia cell lines (THP-1 and U-937); Burkitt lymphoma cell lines (RAJI and NAMALWA); chronic myelogenous leukemia cell lines (NALM1 and K562); multiple myeloma (MM) cell lines (ARH-77, U-266, KHM-11, MIK-1 and RPMI-8226); B-cell lymphoma cell line, T-cell lymphoma cell line and erythroleukemia cell line (BHL-89, HuT-102 and HEL, respectively); and adult T-cell leukemia (ATL) cell line (HPB-MLT). All these cell lines were maintained and cultured in a RPMI 1640 medium (Gibco BRL) containing 10% fetal calf serum (FCS).

The expression of p56^{lck} proteins, SART-1 and SART-2 in each cell line was detected by the Western blot analysis using anti-p56^{lck} protein monoclonal antibody (Lck3A5, Santa Cruz Biotec), anti-SART-1 polyclonal antibody and anti-SART-2 polyclonal antibody. Further, the expression of p56^{lck} protein and SART-3 was analyzed by flow cytometry using Cell Quest software (Becton Dickinson). Intracellular staining was conducted by pre-treating the cells with 4% paraformaldehyde and 0.1 % saponin, and then adding anti-p56^{lck} protein monoclonal antibody and anti-SART-3 monoclonal antibody. FITC-conjugated anti-mouse IgG antibody (ICN Biomedicals) was used as a secondary antibody.

The two different types of lck gene promoters are known, and the type I and the type II are primarily used in tumor cells and normal peripheral blood mononuclear cells, respectively (Non-Patent Reference 12). Then, two lck gene promoters were detected by RT-PCR using specific primer pairs in accordance with the conventional method (Non-Patent Reference 12). Total RNA used as a sample was extracted from cells using RNA-Bee (TEL-TEST). The cDNA was prepared using the Superscript first-strand synthesis systems (Invitrogen). PCR was conducted using Taq DNA polymerase in a DNA cycler (iCycler, Bio-Rad Laboratories) for 30 cycles (at 94°C for I min, at 60°C for 2 min and 72°C for 1 min).

The mRNA expression of ART-1 gene was examined by Northern blot analysis in accordance with the conventional method (Non-Patent Reference 6). human β-actin cDNA (Clontech) was used as a control probe.

The expression of p56^{lck} protein in HPB-MLT leukemia cell line and ARH-77 myeloma cell line as well as in PBMCs and PHA-blastoid T-cells (Figure 1-A) was observed in Western blot analysis. The analysis of lck gene promoters revealed that HPB-MLT, PEER and ML-2 used both promoters, whereas BALL-1, BHL-89 and RAJI used only the type I promoter (Figure 1-B). Thus, it was revealed that most of hematologic malignant cell lines used in the study expressed p56^{lck} mRNAs.

All of SART-1, SART-2 and SART-3 were expressed in hematologic malignant cell lines tested, whereas the expression thereof was not observed in PBMCs.

ART-1 was expressed in all tested hematologic malignant cell lines and PBMCs, however, the expression thereof in most cell lines was higher than that in PBMCs.

The results of the expression study of these proteins in hematologic malignant lines are shown in Table 1. In the table, NT indicates that study was not conducted.

**Table 1**

| Sample | SART-1 | SART-2 | SART-3 | ART-1 | Lck | Lck (type I / type II) | | | |
|---|---|---|---|---|---|---|---|---|---|
| | | | | | | -/- | +/- | -/+ | +/+ |
| ATL | 1/1 | 1/1 | 1/1 | NT | 1/1 | 0/1 | 0/1 | 0/1 | 1/1 |
| T-ALL | 1/1 | 2/2 | 7/7 | 2.0 | 2/2 | 0/2 | 0/2 | 0/2 | 2/2 |
| B-ALL | 2/2 | 4/4 | 6/6 | NT | 1/2 | 0/2 | 2/2 | 0/2 | 0/2 |
| B-lymphoma | 1/1 | 1/1 | 1/1 | NT | NT | 0/1 | 1/1 | 0/1 | 0/1 |
| Plasmocytoma | 5/5 | 5/5 | 2/2 | 2.8 | 3/5 | 1/4 | 2/4 | 0/4 | 1/4 |
| AML | 2/2 | 5/5 | 7/7 | 1.4 | 1/5 | 3/4 | 0/4 | 0/4 | 1/4 |
| CLL | 1/1 | NT | 2/2 | NT | NT | NT | NT | NT | NT |
| CML | NT | NT | 2/2 | 4.6 | NT | NT | NT | NT | NT |
| Burkitt lymphoma | NT | 2/2 | 1/1 | 2.8 | 2/2 | 0/2 | 2/2 | 0/2 | 0/2 |
| PBMCs | 0/2 | 0/2 | 2/2 | 1.0 | 2/2 | 0/2 | 0/2 | 2/2 | 0/2 |
| PHA blastoid | 2/2 | 0/2 | 2/2 | NT | 2/2 | 0/2 | 0/2 | 2/2 | 0/2 |

### EXAMPLE 3

(Induction of cytotoxic T lymphocytes by epithelial cancer-related antigen peptides from PBMCs of a patient with hematologic malignancy)
The induction of peptide-specific cytotoxic T lymphocytes from PBMCs of a patient with hematologic malignancy was conducted as reported preveously (Non-Patent Reference 5).

The following peptides were used to induce cytotoxic T lymphocytes: p56^{lck} protein-derived peptides (Lck₂₀₈ (SEQ ID NO: 1), Lck₄₈₈ (SEQ ID NO: 2) and Lck₄₈₆ (SEQ ID NO: 3)); SART-1-derived peptide (SART-1₆₉₀ (SEQ ID NO: 4)); SART-2-derived peptides (SART-2₉₃ (SEQ ID NO: 5), SART-2₁₆₁ (SEQ ID NO: 6) and SART-2₈₉₉ (SEQ ID NO: 7)); SART-3-derived peptides (SART-3₁₀₉ (SEQ ID NO: 8) and SART-3₃₁₅ (SEQ ID NO: 9)); and ART-1-derived peptide (ART-1₁₇₀ (SEQ ID NO: 10)). All of these peptides are the epithelial cancer-related antigen-derived peptides and have the ability to induce cytotoxic T lymphocytes from PBMCs of an epithelial cancer patient in an HLA-A24 dependent manner (Non-Patent References 3-6 and 12). A human immunodeficiency virus (HIV)-derived peptide (SEQ ID NO: 11) and Epstein-Barr virus (EBV)-derived peptide (SEQ ID NO: 12) were used respectively as a negative control peptide and positive control peptide both of which have an HLA-A24-binding motif. All peptides (> 95% purity) were purchased from Sawady Laboratory, and dissolved respectively in dimethyl sulfoxide at a concentration of 10 mg/ml for use.

PBMCs were prepared from peripheral blood that was collected from a patient with hematologic malignancy using Ficoll-Conray density-gradient centrifugation. The expression of HLA-A24 molecules on PBMCs was determined using flow cytometry by a well known method (Non-Patent Reference 5).

The induction of cytotoxic T lymphocytes from PBMCs was conducted as follows. First, PBMCs (1 x 10⁵ cells/well) were incubated with 10 µM of each peptide in 200 µl of culture medium in a U-bottom-type 96-well microculture plate (Nunc). The culture medium consisted of 45% RPMI-1640, 45% AIM-V (Invitrogen), 10% FCS, 100 U/ml of interleukin-2 (IL-2), and 0.1 mM MEM non-essential amino acid solution (Invitrogen). On days 3^{rd}, 6^{th} and 9^{th} of cultivation, half of the culture medium was removed and replaced with fresh medium containing a corresponding peptide (20 µg/ml). On day 12^{th} of cultivation, the harvested cells were tested for their ability to produce IFN-γ in response to HLA-A24-expressing C1R cells (referred to as CIR-A24) previously pulsed with a corresponding peptide or with an HIV-derived peptide (SEQ ID NO: 11) used as a negative control. The background IFN-γ production in response to the HIV-derived peptide (SEQ ID NO: 11) was subtracted from the values given in the data. When the mean value of the IFN-γ produced from the peptide-stimulated PBMCs in response to a corresponding peptide was higher than that produced in response to the HIV peptide (SEQ ID NO: 11), it was judged that the precursor of a peptide-specific cytotoxic T lymphocyte was present in the PBMCs and that the peptide-specific cytotoxic T lymphocytes were induced by the peptide.

PBMCs of ten cases of HLA-A24⁺ patients with hematologic malignancies (two cases with CLL, one case with MCL, one case with NHL, one case with B-ALL, one case with MM, one case with MDS, two cases with ATL, and one case with T-ALL) were tested for the induction of cytotoxic T lymphocytes using peptides that were derived from p56^{lck} protein, SART-1, SART-2, SART-3 and ART-1. The peptides used were the peptides that induced peptide-specific tumor-reactive cytotoxic T lymphocytes from PBMCs of an HLA-A24⁺ epithelial cancer patient, which are currently on clinical trials for use as vaccines for epithelial cancer patients. The PBMCs were stimulated with the peptide in vitro to measure the peptide-specific IFN-γ production. The results are shown in Table 2.

**Table 2**

| Peptide | SEQ ID NO: | Patient | | | | | | | | | | Responding case /total |
|---|---|---|---|---|---|---|---|---|---|---|---|---|
| | | #1CLL | #2 CLL | #3 MCL | #4 NHL | #5 B-ALL | #6 MM | #7 MDS | #8 ATL | #9 ATL | #10 T-ALL | |
| SART-1₆₉₀ | 4 | 0 | 486 | 0 | 53 | 8 | 141 | 0 | 55 | 155 | 26 | 3/10 |
| SART-2₉₃ | 5 | 0 | 440 | 9 | 63 | 20 | 28 | 0 | 0 | 354 | 192 | 3/10 |
| SART-2₁₆₁ | 6 | 0 | 177 | 49 | 13 | 0 | 28 | 0 | 0 | 198 | 227 | 3/10 |
| SART-2 ₈₉₉ | 7 | 0 | 15 | 12 | 220 | 0 | 0 | 0 | 12 | 346 | 526 | 3/10 |
| SART-3₁₀₉ | 8 | 49 | 0 | 7 | 0 | 0 | 0 | 11 | 49 | 0 | 0 | 0/10 |
| SART-3₃₁₅ | 9 | 39 | 137 | 6 | 0 | 11 | 0 | 0 | 22 | 60 | 6 | 1/10 |
| Lck₂₀₈ | 1 | 0 | 2 | 22 | 30 | 0 | 0 | 0 | 0 | 445 | 153 | 2/10 |
| Lck₄₈₆ | 3 | 0 | 0 | 0 | 0 | 0 | 0 | 0 | 140 | 2099 | 140 | 3/10 |
| Lck₄₈₈ | 2 | 0 | 0 | 0 | 0 | 6 | 182 | 102 | 476 | 304 | 0 | 4/10 |
| ART-1₁₇₀ | 10 | 501 | 33 | 0 | 0 | 0 | 94 | 0 | 69 | 1487 | 3 | 2/10 |
| HIV | 11 | 0 | 71 | 8 | 0 | 0 | 0 | 9 | 0 | 0 | 2 | 0/10 |
| EBV | 12 | 0 | 100 | 0 | 100 | 0 | 418 | 115 | 264 | 264 | 0 | 6/10 |

An enhancement of IFN-γ production from PBMCs stimulated with Lck₂₀₈ (SEQ ID NO: 1), Lck₄₈₆ (SEQ ID NO: 3) and Lck₄₈₈ (SEQ ID NO: 2) were observed in two, three and four out of ten cases respectively. Lck₂₀₈ (SEQ ID NO: 1) enhanced IFN-γ production from PBMCs of one case of ATL and one case of T-ALL. Lck₄₈₆ (SEQ ID NO: 3) enhanced the IFN-γ production from PBMCs of two cases of ATL and one case of T-ALL. Lck₄₈₈ (SEQ ID NO: 2) enhanced the IFN-γ production from PBMCs of one case of MM, one case of MDS, and two cases of ATL. On the other hand, the stimulation of PBMCs with the positive control, EBV derived peptides (SEQ ID NO: 12), showed an enhancement of IFN-γ production in six out of ten cases, while the stimulation with the negative control, HIV-derived peptide (SEQ ID NO: 11), did not induce IFN-γ production in any case.

PBMCs in the aforementioned studies which showed the enhanced IFN-γ production by the incubation with the peptide in response to CIR-A24 pulsed with the peptide, were further incubated for two weeks in the presence of IL-2 (100 U/mL) alone. Then, the cytotoxicity against hematologic malignant cell lines was examined by a standard ⁵¹Cr-release test (Non-Patent Reference 5). Specifically, the PBMCs obtained were used as an effector to mix with ⁵¹Cr-labelled target cells (hereinafter referred to as targets) at an effector/target rate of 3, 10 or 30, and then incubated for six hours, followed by measuring the radioactivity of ⁵¹Cr released in the supernatants. The results obtained were indicated as % lysis which was calculated by regarding a radioactivity as 100% when the targets were completely dissolved. The following targets were used: PEER, an HLA-A24⁺ hematologic malignant cell line; HPB-MLT, an HLA-A24⁻ hematologic malignant cell line; and an HLA-A24⁺ PHA blastoid T-cell.

The representative results are shown in Figures 2-A, 2-B and 2-C. PBMCs, which were stimulated with Lck₄₈₆ (SEQ ID NO: 3), SART-2₁₆₁ (SEQ ID NO: 6) or SART-2₈₉₉ (SEQ ID NO: 7) and further cultured in the presence of IL-2 (patient Nos. 8, 2, and 4 respectively in table 2), showed significant cytotoxic activity against an HLA-A24⁺ hematologic malignant line, but did not show cytotoxic activity against an HLA-A24⁻ hematologic malignant line and an HLA-A24⁺PHA blastoid T-cells (Figures 2-A, 2-B, and 2-C respectively). Such cytotoxic activity was not observed in PBMCs cultured with the HIV-derived peptide (SEQ ID NO: 11) or EBV derive peptides (SEQ ID NO: 12).

It was revealed that Lck₄₈₆ (SEQ ID NO: 3), SART-2₁₆₁ (SEQ ID NO: 6) and SART-2₈₉₉ (SEQ ID NO: 7) had the abilities to induce tumor-specific cytotoxic T lymphocytes from PBMCs of a patient with hematologic malignancy in an HLA-A24 dependent manner.

### EXAMPLE 4

(Vaccination of cancer vaccine to a patient with hematologic malignancy: Study 1)
The patient (patient No. 6 in table 2) subjected to clinical trials was a 74-year old HLA-A24⁺ male suffering from IgA-type multiple myeloma with a stage III by Durie & Salmon's classification. The patient received multiple cycles of chemotherapy before participating in this clinical test and had acquired the resistance to chemotherapy. However, for four weeks before peptide vaccination, the patient had not received chemotherapy and administration with steroid or any other immunosuppressive drugs.

The expression of HLA-A24 molecules on CD38⁺ multiple myeloma cells (MM cells) that was derived from the patient's bone marrow was confirmed by flow cytometry with anti-HLA-A24 monoclonal antibody. Further, it was confirmed by the same way using anti-Lck antibody that these CD38⁺ MM cells expressed Lck protein.

The peptides used in vaccination were Lck₄₈₈ (SEQ ID NO: 2), SART-1₆₉₀ (SEQ ID NO: 4) and ART-1₁₇₀ (SEQ ID NO: 10). These peptides had induced cytotoxic T lymphocytes in vitro from PBMCs derived from the pre-vaccinated patient. Therefore, it was believed that there existed in the patient's PBMCs peptide-specific cytotoxic T lymphocyte precursors reactive to these peptides.

Three kinds of emulsions each of which contains each of these peptides respectively and an adjuvant were prepared according to the method described in Example 1, and vaccinated to the patient by injecting subcutaneously at different sites of the patient's lateral thigh. The vaccination was conducted after confirming by skin test that the patient showed no immediate hypersensitivity reaction against the peptides. The skin test was conducted by injecting intradermally the solution of 50 µg of each peptide in physiological saline and determining immediate-type or delayed-type hypersensitivity reaction at 20 minutes or 24 hours after the injection.

The vaccinations were conducted seven times every two weeks using SART-1₆₉₀ (SEQ ID NO: 4), Lck₄₈₈ (SEQ ID NO: 2) and ART-1₁₇₀ (SEQ ID NO: 10). The cytotoxic activity of PBMCs acquired after the sixth vaccination was measured according to the in vitro test described in Example 3. As a result, the peptide-specific cytotoxic activity against SART-1₆₉₀ (SEQ ID NO: 4), Lck₄₈₈ (SEQ ID NO: 2) and Lck₂₀₈ (SEQ ID NO: 1) respectively was observed in the PBMCs. Therefore, in the eighth and later vaccinations, three kinds of emulsions each of which contains SART-1₆₉₀ (SEQ ID NO: 4), Lck₄₈₈ (SEQ ID NO: 2) or Lck₂₀₈ (SEQ ID NO: 1) and an adjuvant were prepared according to the method described in Example 1 and used.

After the vaccinations, PBMCs derived from the patient were studied for their peptide-specific cytotoxic activity by ⁵¹Cr-release test using C1R-A24 cells pulsed with the peptides used. The results revealed that the peptide-specific cytotoxic activity was enhanced with the increasing number of vaccination (Figure 3-A). When PBMCs were collected after the 3^{rd}, 6^{th} and 9^{th} vaccination and tested for their cytotoxic activity against myeloma cell line, the PBMCs showed significant levels of cytotoxic activity against KHM-11 and MIK-1 both of which are HLA-A24⁺ myeloid cell lines (Figure 3-B). On the other hand, their cytotoxic activity was extremely low against RPMI-8226 that is an HLA-A24⁻ myeloid cell line. The cytotoxic activity was inhibited significantly by anti-HLA class I antibody (W6/32, IgG2a), anti-CD8 antibody (Nu-Ts/c, IgG2a) or anti-HLA-A24 antibody (A11.1, IgG3), but not inhibited by anti-CD 14 antibody (JML-H14, IgG2a).

Thus, it was revealed that the peptide-vaccination induced HLA-A24-restricted tumor-specific cytotoxic T lymphocytes in the blood of a patient with multiple myeloma.

With regard to the clinical responses, examination of bone marrow revealed that the plasma cells showed 46% reduction (from 46.6 x 10⁴/µl in pre-vaccination to 21.5 x 10⁴/µl after the sixth vaccination), and stable conditions has been kept for more than nine months.

With regard to the side effects, the local reactions of vaccinations were observed only at the sites of administration (grade II) to such a level that no treatment was needed for them. Delayed-type hypersensitivity reaction was not observed against the peptides used in the vaccination. In this case, no symptom indicating autoimmune reactions was observed.

Thus, it was revealed that the vaccination of the aforementioned peptides was effective for treating hematologic malignancies with very few side effects.

### EXAMPLE 5

(Peptide preparation used for vaccination: 2)
Two kinds of emulsions were prepared using SART-2₉₃ (SEQ ID NO: 5) that is a SART-2-derived peptide and SART-3₁₀₉ (SEQ ID NO: 8) that is a SART-3-derived peptide by the same method with the same adjuvant as in Example 1.

### EXAMPLE 6

(Vaccination of cancer vaccine to a patient with hematologic malignancy: Study 2)
The patient (patient No. 1 in table 2) subjected to clinical trials was a 63-year old HLA-A24⁺ female suffering from chronic lymphocytic leukemia with a stage A by Bernet's classification. The patient showed progressively increasing white blood cell counts and decreasing platelet counts at the start of this clinical trial.

The expression of HLA-A24 molecules on CD19⁺ chronic lymphocytic leukemia cells (CLL cells) that was derived from the patient's PBMCs was confirmed by flow cytometry with anti-HLA-A24 monoclonal antibody.

ART-1₁₇₀ (SEQ ID NO: 10) induced cytotoxic T lymphocytes in vitro from PBMCs of the pre-vaccinated patient, however it caused immediate hypersensitivity reaction in skin test (see Example 4) conducted before vaccination. Therefore, SART-2₉₃ (SEQ ID NO: 5) and SART-3₁₀₉ (SEQ ID NO: 8) were used for vaccination. Since significant levels of Immunoglobulin G antibody reactive to these peptides were detected in a pre-vaccinated serum, it was suggested that humoral immunity against these peptides had been evoked in the patient before vaccination. In the clinical trials of peptide vaccines in epithelial cancer patients, there has been observed a correlation between the survival rate of patients and the increased humoral immune responses against the peptides used in the vaccines. From these facts, it was believed that SART-2₉₃ (SEQ ID NO: 5) and SART-3₁₀₉ (SEQ ID NO: 8) were effective in this patient.

Two kinds of emulsions prepared using these peptides according to the methods described in Example 5 were vaccinated to the patient by injecting subcutaneously at different sites of the patient's lateral thigh.

After each vaccination, PBMCs derived from the patient were studied for the peptide-specific cytotoxic T lymphocyte induction by measuring the amount of IFN-γ production as an indicator against C1R-A24 cells pulsed with the peptides used (see Example 3). The results revealed that the amount of IFN-γ production increased with the increasing number of vaccination (Figure 4-A). The IFN-γ production from PBMCs was significantly inhibited by anti-CD8 antibody (Nu-Ts/c, IgG2a) or anti-HLA-A24 antibody (A11.1, IgG3), but was not inhibited by anti-CD14 antibody (JML-H14, IgG2a).

These results revealed that peptide-reactive cytotoxic T lymphocytes were induced by the vaccination. Further, it was revealed that these peptide-reactive cytotoxic T lymphocytes showed higher cytotoxic activity against C1R-A24 cells pulsed with SART-2₉₃ (SEQ ID NO: 5) or SART-3₁₀₉ (SEQ ID NO: 8) than against the cells pulsed with the HIV-derived peptide (SEQ ID NO: 11) in the ⁵¹Cr-release test (Figure 4-B).

Thus, it was revealed that the peptide-vaccination induced HLA-A24-restricted peptide-specific cytotoxic T lymphocytes in the blood of a patient with chronic lymphocytic leukemia.

With regard to the clinical responses, 20 x 10³/µl of the lymphocyte counts in blood before the vaccination decreased significantly to 12.8 x 10³/µl after the sixth vaccination, although it was temporary. The platelet counts were low before the vaccination as it was 10 x 10⁴/µl, which increased after the sixth vaccination and was maintained within the normal ranges through the period of 17 vaccinations.

With regard to the side effects of vaccinations, local reactions were observed at the administration sites to such a level that no treatment was needed (grade I). Further, after the third vaccination, delayed-type hypersensitivity reaction was observed, which indicated that one of the immune response was evoked against SART 3₁₀₉ (SEQ ID NO: 8). Thus, there were very few side effects observed.

### INDUSTRIAL APPLICABILITY

The agent for preventing and/or treating hematologic malignancies according to the present invention is useful because it may be used for a specific immunotherapy of hematologic malignancies, and thus has a very high applicability in the pharmaceutical field.

### SEQUENCE LISTING FREE TEXT

SEQ ID NO: 1: Partial peptide of p56^{lck} protein
SEQ ID NO: 2: Partial peptide of p56^{lck} protein
SEQ ID NO: 3: Partial peptide of p56^{lck} protein
SEQ ID NO: 4: Partial peptide of SART-1 protein
SEQ ID NO: 5: Partial peptide of SART-2 protein
SEQ ID NO: 6: Partial peptide of SART-2 protein
SEQ ID NO: 7: Partial peptide of SART-2 protein
SEQ ID NO: 8: Partial peptide of SART-3 protein
SEQ ID NO: 9: Partial peptide of SART-3 protein
SEQ ID NO: 10: Partial peptide of ART-1 protein
SEQ ID NO: 11: Human Immunodeficiency Virus-derived peptide
SEQ ID NO: 12: Epstein-Barr-Virus-derived peptide

## Claims

1. An agent for preventing and/or treating hematologic malignancies, comprising as an active ingredient at least one peptide having an amino acid sequence selected from the group consisting of SEQ ID NOs: 1, 2, 3, 4, 5, 6, 7, 8, 9 and 10 in the sequence listing.

2. An agent for preventing and/or treating hematologic malignancies, comprising as an active ingredient at least one peptide having an amino acid sequence selected from the group consisting of SEQ ID NOs: 1, 2, 4, 5, 8 and 10 in the sequence listing.

3. The agent for preventing and/or treating hematologic malignancies according to Claim 2, further comprising as an active ingredient at least one peptide having an amino acid sequence selected from the group consisting of SEQ ID NOs: 3, 6, 7 and 9 in the sequence listing.

4. An agent for preventing and/or treating hematologic malignancies, comprising as an active ingredient at least one peptide having an amino acid sequence selected from the group consisting of SEQ ID NOs: 1, 2 and 4 in the sequence listing.

5. An agent for preventing and/or treating hematologic malignancies, comprising as an active ingredient at least one peptide having an amino acid sequence selected from the group consisting of SEQ ID NOs: 2, 4 and 10 in the sequence listing.

6. An agent for preventing and/or treating hematologic malignancies, comprising as an active ingredient at least one peptide having an amino acid sequence selected from the group consisting of SEQ ID NOs: 5 and 8 in the sequence listing.

7. The agent for preventing and/or treating hematologic malignancies according to any one of Claims 1 to 6, further comprising an adjuvant.

8. The preventing and/or treating agent according to any one of Claims 1 to 7, wherein the hematologic malignancies are hematologic malignancies having HLA-A24 molecules on their cell surface.

9. The preventing and/or treating agent according to any one of Claims 1 to 7, wherein the hematologic malignancies are hematologic malignancies which have HLA-A24 molecules on their cell surface and express a protein containing the peptide that is an active ingredient for the preventing and/or treating agent.

10. The agent for preventing and/or treating hematologic malignancies according to any one of Claims 1 to 9, wherein the agent is used as a cancer vaccine for the hematologic malignancies.

11. A medicament for preventing and/or treating hematologic malignancies having HLA-A24 molecules on their cell surface, comprising at least one peptide having an amino acid sequence selected from the group consisting of SEQ ID NOs: 1, 2, 4, 5, 8 and 10 in the sequence listing and an adjuvant.

12. A method for preventing and/or treating hematologic malignancies that have HLA-A24 molecules on their cell surface, comprising administering a therapeutically effective amount of at least one peptide having an amino acid sequence selected from the group consisting of SEQ ID NOs: 1, 2, 4, 5, 8 and 10 in the sequence listing together with an adjuvant.

## Amended claims

### Amended claims under Art. 19.1 PCT

1. An agent for preventing and/or treating hematologic malignancies, comprising as an active ingredient at least one peptide having an amino acid sequence selected from the group consisting of SEQ ID NOs: 1, 2, 3, 4, 5, 6, 7, 8, 9 and 10 in the sequence listing.

2. An agent for preventing and/or treating hematologic malignancies, comprising as an active ingredient at least one peptide having an amino acid sequence selected from the group consisting of SEQ ID NOs: 1, 2, 4, 5, 8 and 10 in the sequence listing.

3. The agent for preventing and/or treating hematologic malignancies according to Claim 2, further comprising as an active ingredient at least one peptide having an amino acid sequence selected from the group consisting of SEQ ID NOs: 3, 6, 7 and 9 in the sequence listing.

4. An agent for preventing and/or treating hematologic malignancies, comprising as an active ingredient at least one peptide having an amino acid sequence selected from the group consisting of SEQ ID NOs: 1, 2 and 4 in the sequence listing.

5. An agent for preventing and/or treating hematologic malignancies, comprising as an active ingredient at least one peptide having an amino acid sequence selected from the group consisting of SEQ ID NOs: 2, 4 and 10 in the sequence listing.

6. An agent for preventing and/or treating hematologic malignancies, comprising as an active ingredient at least one peptide having an amino acid sequence selected from the group consisting of SEQ ID NOs: 5 and 8 in the sequence listing.

7. The agent for preventing and/or treating hematologic malignancies according to any one of Claims 1 to 6, further comprising an adjuvant.

8. The preventing and/or treating agent according to any one of Claims 1 to 7, wherein the hematologic malignancies are hematologic malignancies having HLA-A24 molecules on their cell surface.

9. The preventing and/or treating agent according to any one of Claims 1 to 7, wherein the hematologic malignancies are hematologic malignancies which have HLA-A24 molecules on their cell surface and express a protein containing the peptide that is an active ingredient for the preventing and/or treating agent.

10. The agent for preventing and/or treating hematologic malignancies according to any one of Claims 1 to 9, wherein the agent is used as a cancer vaccine for the hematologic malignancies.

11. A medicament for preventing and/or treating hematologic malignancies having HLA-A24 molecules on their cell surface, comprising at least one peptide having an amino acid sequence selected from the group consisting of SEQ ID NOs: 1, 2, 4, 5, 8 and 10 in the sequence listing and an adjuvant.

12. Use of a therapeutically effective amount of at least one peptide having an amino acid sequence selected from the group consisting of SEQ ID NOs: 1, 2, 4, 5, 8 and 10 in the sequence listing together with an adjuvant for the production of a pharmaceutical composition for preventing and/or treating hematologic malignancies that have HLA-A24 molecules on their cell surface.


SEQUENCE LISTING

<110>  ITOH, Kyogo

<120>  Composition for preventing and/or treating hematological malignancy

<130>  GP04-1014PCT

<150>  JP P2003-287208
<151>  2003-08-05

<160>  12

<170>  PatentIn version 3.1

<210>  1
<211>  9
<212>  PRT
<213>  Artificial

<220>
<223>  Partial peptide of p56lck protein

<400>  1

His Tyr Thr Asn Ala Ser Asp Gly Leu
1               5


<210>  2
<211>  10
<212>  PRT
<213>  Artificial

<220>
<223>  Partial peptide of p56lck protein

<400>  2

Asp Tyr Leu Arg Ser Val Leu Glu Asp Phe
1               5                   10


<210>  3
<211>  9
<212>  PRT
<213>  Artificial

<220>
<223>  Partial peptide of p56lck protein

<400>  3

Thr Phe Asp Tyr Leu Arg Ser Val Leu
1               5


<210>  4

<211>  9
<212>  PRT
<213>  Artificial

<220>
<223>  Partial peptide of SART-1

<400>  4

Glu Tyr Arg Gly Phe Thr Gln Asp Phe
1               5


<210>  5
<211>  9
<212>  PRT
<213>  Artificial

<220>
<223>  Partial peptide of SART-2

<400>  5

Asp Tyr Ser Ala Arg Trp Asn Glu Ile
1               5


<210>  6
<211>  9
<212>  PRT
<213>  Artificial

<220>
<223>  Partial peptide of SART-2

<400>  6

Ala Tyr Asp Phe Leu Tyr Asn Tyr Leu
1               5


<210>  7
<211>  9
<212>  PRT
<213>  Artificial

<220>
<223>  Partial peptide of SART-2

<400>  7

Ser Tyr Thr Arg Leu Phe Leu Ile Leu
1               5


<210>  8
<211>  10
<212>  PRT

<213>  Artificial

<220>
<223>  Partial peptide of SART-3

<400>  8

Val Tyr Asp Tyr Asn Cys His Val Asp Leu
1               5                   10


<210>  9
<211>  9
<212>  PRT
<213>  Artificial

<220>
<223>  Partial peptide of SART-3

<400>  9

Ala Tyr Ile Asp Phe Glu Met Lys Ile
1               5


<210>  10
<211>  9
<212>  PRT
<213>  Artificial

<220>
<223>  Partial peptide of ART-1

<400>  10

Glu Tyr Cys Leu Lys Phe Thr Lys Leu
1               5


<210>  11
<211>  11
<212>  PRT
<213>  Artificial

<220>
<223>  Human Immunodeficiency Virus-derived peptide

<400>  11

Arg Tyr Leu Arg Asp Gln Gln Leu Leu Gly Ile
1               5                   10


<210>  12
<211>  9
<212>  PRT
<213>  Artificial

<220>
<223>  Epstein-Barr-Virus-derived peptide

<400>  12

Thr Tyr Gly Pro Val Phe Met Cys Leu
1               5